# EUROPEAN PATENT APPLICATION

(11) **EP 4 446 981 A1**
(43) Date of publication of application: **16.10.2024**
(21) Application number: 23305554.0
(22) Date of filing: 13.04.2023
(51) Int. Cl.: G06T 7/00, G06T 7/11

(54) **STANDARD PLANE DETECTION IN A MEDICAL IMAGE**

(71) Applicant: Sonio, 75014 Paris (FR)
(72) Inventor: BESSON, Rémi, 75014 PARIS (FR); LOGE MUNEREL, Frédéric, 75014 PARIS (FR); Debavelaere, Vianney, 75014 PARIS (FR)
(74) Representative: Plasseraud IP

(57) **Abstract**

Method for determining a standard plane in a medical image, said method comprising: determining (S10) at least one region of interest in the medical image, determining (S11) at least one candidate plane of said region of interest, determining (S12) at least one anatomical structure in the region of interest or in the medical image, selecting (S13) a plane from the group of said candidate planes based on the presence or the absence of said anatomical structure in said candidate plane, said selected plane being the standard plane.

## Description

### Technical Field

1. This disclosure pertains to the field of medical images. In particular, the invention relates to a method for providing decision support for medical imaging by determining a standard plane in a medical image.

### Background Art

2. The field of medical imaging includes numerous imaging modalities aiming to provide various images of parts of the body for diagnostic or treatment purposes.

3. Generally speaking, a large amount of images is acquired by a specialist, for instance by a physician, during a medical examination using medical imaging. Said large amount of images is manually reviewed by the specialist during the acquisition so as to assess whether or not the necessary measurements have been acquired.

4. Therefore, current review of the acquired dataset is highly dependent on specialists.

### Summary

5. This disclosure improves the situation.

6. More specifically, it is proposed a method for determining a standard plane in a medical image, said method comprising:
- determining at least one region of interest in the medical image,
- determining at least one candidate plane of said region of interest,
- determining at least one anatomical structure in the region of interest or in the medical image,
- selecting a plane from the group of said candidate planes based on the presence or the absence of said anatomical structure in said candidate plane, said selected plane being the standard plane.

7. The region of interest may designate a part or the entirety of the medical image. The region of interest may comprise an element of interest. In one or more embodiments, the element of interest may be chosen in a predefined group specific to the medical imaging modality and/or to the medical examination. For instance, for a fetal ultrasound image, the element of interest may be an element in the group comprising: a brain, a vessel, an orbit, a nose, a mouth, a cavity, an abdomen etc.

8. The region of interest may be manually or automatically determined. In one or more embodiments, the region of interest may be a bounded box comprising the element of interest. More specifically, in some examples, the region of interest may be the minimum bounded box comprising the element of interest.

9. The candidate plane may designate a reference plane. The reference plane may depend on the medical imaging modality and/or the medical examination. The reference plane may be chosen in a group specific to the medical imaging modality and/or to the medical examination. For instance, for a fetal ultrasound image, the reference plane may be a view in the group comprising the following views: a transthalamic view, a transcerebellar view, a transventricular view, an upper lip nose and nostrils view, an orbits view, a coronal view of the thorax-abdomen, a four chambers view of the heart, a left ventricular outflow tract, a right ventricular outflow tract, a three vessels view of the heart, a spine view, a transverse section of the abdomen, a bladder view, a femur diaphysis length view, a view of the two kidneys, a crown rump length (CRL) view, a nuchal translucency view, a hands view, a feet view, a profile view, a cervix length view, a 3D view and a Doppler view.

10. The standard plane may designate the reference plane, if any, associated by the proposed method with the medical image.

11. Advantageously, the proposed method allows to identify whether an acquired medical image corresponds to a standard plane. Hence, a specialist acquiring the medical image may be provided with a verification that the acquired medical image corresponds to a standard plane and even to an identification of said standard plane. In particular, in one or more embodiments, an alert may be triggered based on the presence or the absence of a given anatomical structure in the medical image. More specifically, in some embodiments, the alert may be triggered when a given anatomical structure associated with the determined standard plane is not present in the medical image. For instance, when the transthalamic view is determined in the medical image and the cavum septum pellicidum is absent in the medical image, the alert may be triggered to warn the specialist about this inconsistency.

12. Further, the proposed method allows to reduce the number of errors in the identification of the standard plane by selecting the standard plane from the group of candidate planes based on the presence or the absence of the detected anatomical structure in the candidate plane. That is, the proposed method refines the determination of the at least one candidate plane based on the determination of the at least one anatomical structure in the region of interest or in the medical image. The determination of the at least one anatomical structure in the region of interest or in the medical image may hence allow to correct errors resulting from the determination of the at least one candidate plane.

13. The following features, can be optionally implemented, separately or in combination one with the others.

14. In one or more embodiments, the proposed method may be iterated for a plurality of medical images. In particular, the proposed method may be iterated until all standard planes in a group of standard planes are determined. Further, a list comprising the standard planes for which no medical image has been acquired may be provided to the specialist performing the medical examination. Hence, the specialist may be guided through the medical examination to ensure that all medical images specific to a medical examination are obtained.

15. In one or more embodiments, a first alert may be triggered based on the one or more determined standard planes. For instance, the alert may be triggered when the list comprising the standard planes for which no medical image has been acquired comprises one or more elements at the end of the medical examination. That is, the alert may be triggered when a medical image for at least one standard plane is missing. In particular, the alert may be triggered at the end of the medical examination to warn the specialist of one or more missing standard planes.

16. Alternatively or in combination, a second alert may be triggered based on a quality of the one or more medical images. For instance, the second alert may be triggered when the quality of at least one medical image is lower than a quality threshold. In one or more embodiments, for a plurality of medical images having a quality lower than the quality threshold, or even for each medical image having a quality lower than the quality threshold, a respective second alert may be triggered.

17. Alternatively or in combination, a third alert may be triggered based on the presence or the absence of a given anatomical structure in the medical image. More specifically, in some embodiments, the third alert may be triggered when a given anatomical structure associated with the determined standard plane is not present in the medical image. For instance, when the transthalamic view is determined in the medical image and the cavum septum pellicidum is absent in the medical image, the third alert may be triggered to warn the specialist about this inconsistency.

18. In one or more embodiments, the first and/or second alerts and/or third alerts may be triggered simultaneously. In particular, in one or more embodiments, the first, second and third alerts may be triggered simultaneously at the end of the medical examination. Alternatively, in one or more embodiments, the second alerts may be triggered upon determination of the quality of the medical image lower than the quality threshold. Further, in one or more embodiments, the third alert may be triggered upon detection of the presence and/or the absence of given anatomical structures in the medical image.

19. In one or more embodiments, the region of interest may be associated with a first confidence score and the determination of the candidate plane of said region of interest may depend on said first confidence score.

20. The first confidence score may designate a probability that the region of interest comprises an element of interest.

21. The determination of the candidate plane may depend on said first confidence score. In one or more embodiments, a threshold of detection may be associated with the region of interest. The candidate plane may be determined if the first confidence score associated with the region of interest is higher than the threshold of detection.

22. Advantageously, in some embodiments, different regions of interest may be associated with different thresholds of detection. For instance, the more specific the region of interest is, the lower the threshold of detection may be. Therefore, a sensitivity of the determination of the candidate plane may be increased.

23. In one or more embodiments, each candidate plane from the group of said candidate planes may be associated with a priority level and/or a second confidence score, and the method may further comprise:
- ranking the candidate planes based on the priority levels and/or the second confidence scores,
and the selection of the plane from the group may be based on the candidate planes' rank order.

20. The priority level may be based on an a *priori* knowledge. The priority level may depend on the medical imaging modality and/or the medical examination. For instance, for a fetal ultrasound image, it may be known that the hand view or the feet view may not be the views of primary importance and may result from a fetal positioning hiding the view of primary importance, for instance the crown rump length view. Hence, the hand view and the feet view may have a lower priority than a crown rump length view.

21. The second confidence score may designate a probability that the candidate plane corresponds to the determined region of interest. In one or more embodiments, the second confidence score may be equal to the first confidence score.

22. Selecting the plane from the group of candidate planes based on the candidate planes' rank order may allow to determine the acquired standard plane even if several regions of interest are detected on the medical image. For instance, in some situations, the specialist may not be able to properly image the standard plane without including one or more regions of interest which are not related to the standard plane. As above-mentioned, for a fetal ultrasound, this situation may occur when the fetus has its hand and/or feet positioned over another region of interest. Advantageously, ranking the candidate planes may allow to disentangle images comprising several regions of interest while allowing to determine the proper standard plane of the medical image.

23. In one or more embodiments, the method may further comprise:
- detecting writings in the region of interest or in the medical image,
- reducing the group of candidate planes based on said detected writings.

24. That is, in one or more embodiments, the group of candidate planes may be determined based on the determined region of interest and on the writings detected in the medical image. For instance, the writings detected in the medical image may outline a contradiction with some of the possible candidate planes. Therefore, a reduced group of candidate planes may be obtained based on the writings detected in the medical image, increasing the accuracy of the standard plane determination. Further, by reducing the group of candidate planes, an increased efficiency of the standard plane detection may be achieved.

25. In one or more embodiments, the method may further comprise:
- determining an apparatus that provided the medical image, reducing the group of candidate planes based on said determined apparatus.

26. Advantageously, the determination of the apparatus that provided the medical image may orientate the standard plane determination. That is, reduction of the group of candidate planes may be apparatus dependent. By reducing the group of candidate planes, an increased efficiency of the standard plane detection may be achieved.

27. In one or more embodiments, the method may further comprise: detecting, based on the apparatus detected, the writings in the region of interest or in the medical image.

28. That is, detection of the writings in the medical image may be orientated based on the determined apparatus. For instance, the knowledge of the apparatus that provided the medical image may reduce the sets of pixels of the medical images considered for the writing detection. In one or more embodiments, the knowledge of the apparatus that provided the medical image may reduce the possible parts of the medical image in which the detection of the writings is performed. For instance, writing detection may be performed in localized areas of the medical images. Alternatively or in combination, writing detection may be performed for sets of pixels having one or more specific colors, said specific colors depending on the determined apparatus. Hence, by orientating the detection of the writings in the medical image, an increased efficiency of the standard plane determination may be achieved.

29. In one or more embodiments, the method may further comprise:
- analyzing colors of pixels present in the region of interest or in the medical image,
- reducing the group of candidate planes based on said colors' analysis.

30. That is, an analysis of the colors, or colorimetry, of pixels present in the region of interest or in the medical image may be used to reduce the group of candidate planes. For instance, fetal ultrasound views, 3D views and Doppler views may be associated with distinct sets of pixels colors. Hence, analyzing the colors of pixels present in the region of interest or in the medical image may allow to discard some planes from the group of possible candidate planes for the medical image. Consequently, by using a colorimetry analysis of the pixels in the medical image, an increased efficiency of the standard plane determination may be achieved.

31. In one or more embodiments, the method may further comprise:
- selecting a plurality of planes based on the presence or the absence of the anatomical structure in the candidate plane,
- displaying the plurality of selected planes,
- receiving information on the selected plane corresponding to the standard plane,
- storing received information in a database, said database being used to train the standard plane determination.

32. In one or more embodiments, the method further comprises respectively associating to said at least one anatomical structure at least one third confidence score.

33. In one or more embodiments, the method further comprises selecting, among a plurality of medical images, an output image based on said at least one third confidence score.

34. In one or more embodiments, the method comprises : computing a plurality of third confidence scores for a plurality of reference anatomical structures respectively; computing a weighted third confidence score as a weighted average of said plurality of third confidence scores.

35. In one or more embodiments, the method comprises: selecting a subset of said plurality of medical images where the highest number of reference anatomical structures are present; selecting said output image as the image of said subset that has the highest weighted third confidence score.

36. That is, in one or more embodiments, a plurality of planes may be selected with a method of the present disclosure. Information may be received to select the standard plane among the plurality of selected planes. For instance, received information may comprise a selection of the standard plane by the specialist. Received information may be stored in the database used to train the standard plane determination. Hence, received information may increase the reliability of the standard plane determination for the current standard plane determination and for future standard plane determinations.

37. The present disclosure also concerns an apparatus for determining a standard plane in a medical image, said apparatus including a computing module adapted to implement at least a part of a method as defined here.

38. In another aspect, it is proposed a computer software comprising instructions to implement at least a part of a method as defined here when the software is executed by a processor. In another aspect, it is proposed a computer-readable non-transient recording medium on which a software is registered to implement the method as defined here when the software is executed by a processor.

### Brief Description of Drawings

39. Other features, details and advantages will be shown in the following detailed description and on the figures, on which:
**Fig. 1**

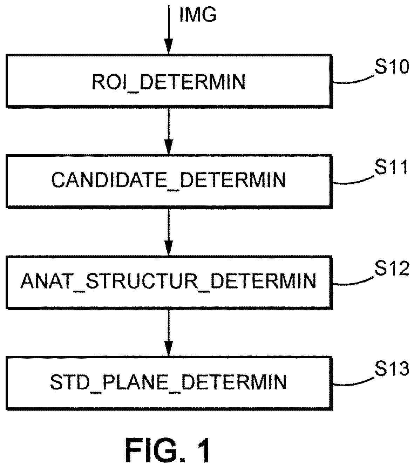

   40. [Fig. 1] is a flowchart illustrating a possible method for determining a standard plane in a medical image according to an embodiment of the present invention.
**Fig. 2**

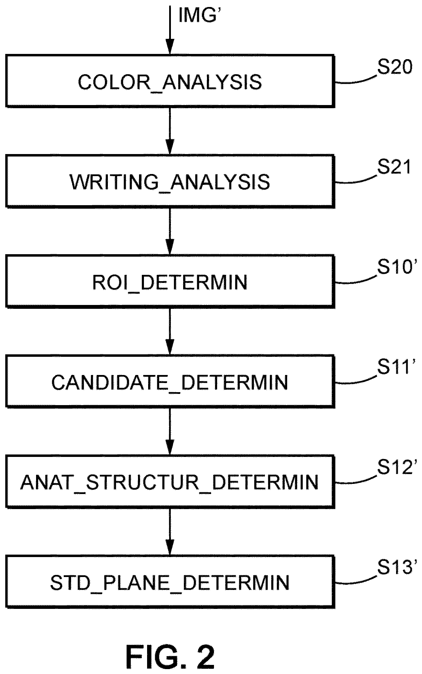

   41. [Fig. 2] is a flowchart illustrating a possible method for determining a standard plane in a medical image according to an embodiment of the present invention.
**Fig. 3**

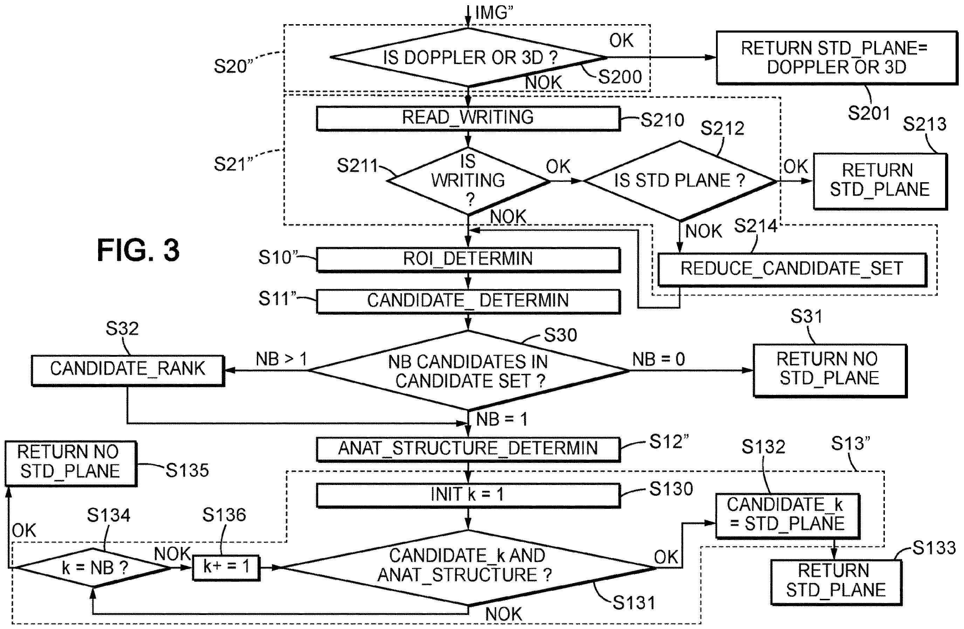

   42. [Fig. 3] is a flowchart illustrating a possible method for determining a standard plane in a medical image according to an embodiment of the present invention.
**Fig. 4**

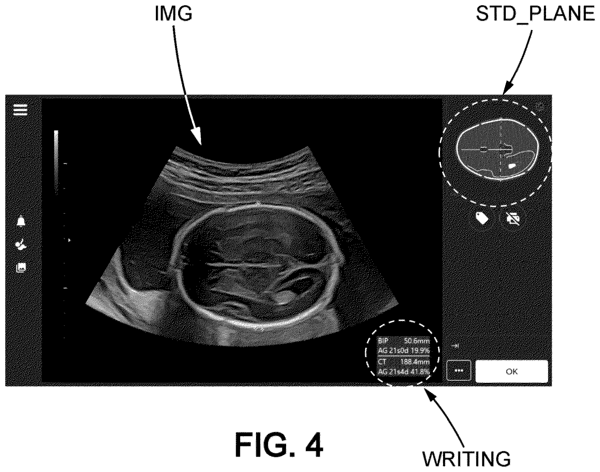

   43. [Fig. 4] shows an example of a standard plane determined with a method of the present disclosure.
**Fig. 5**

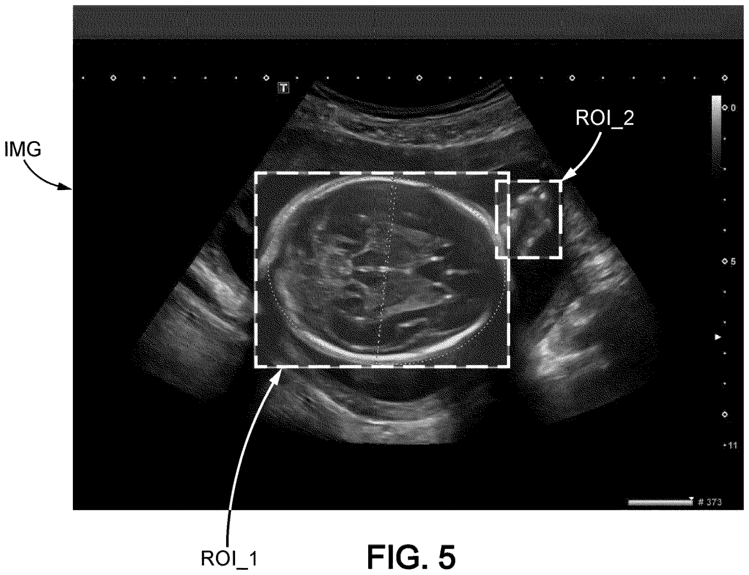

   [Fig. 5] shows an example of a standard plane determined with a method of the present disclosure.
**Fig. 6**

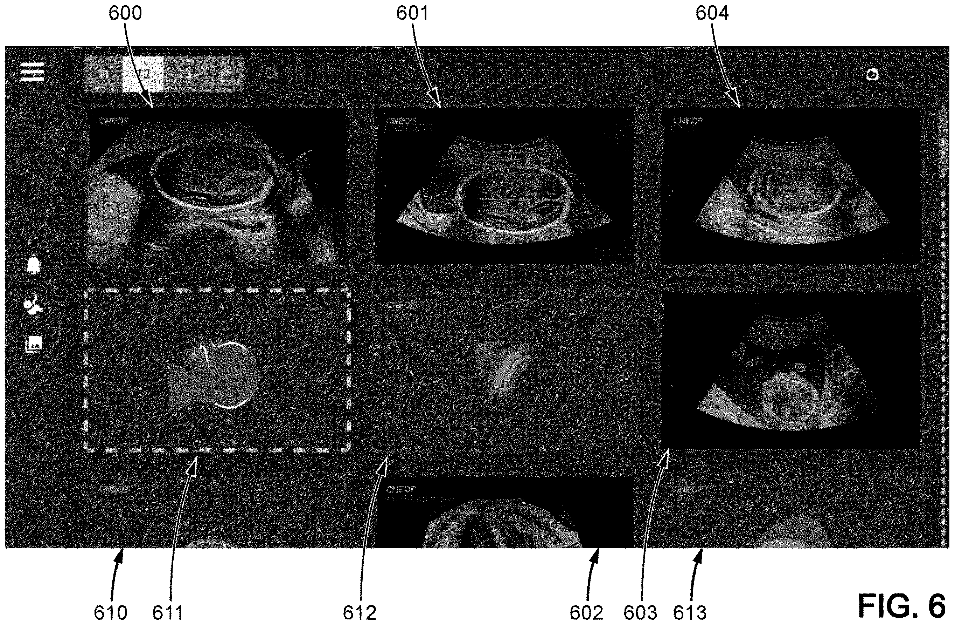

   44. [Fig. 6] shows an example of candidate planes and of standard planes determined with a method of the present disclosure.
**Fig. 7**

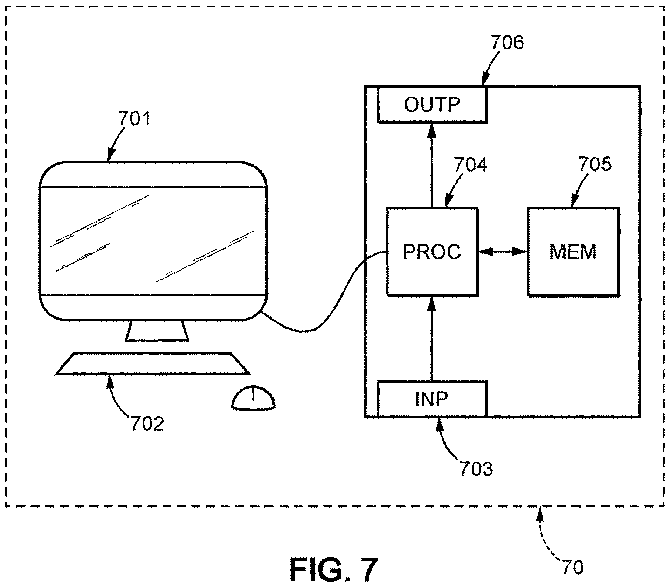

   45. [Fig. 7] is a block diagram illustrating an embodiment of an apparatus according to the present disclosure.

### Description of Embodiments

46. For simplicity and clarity of illustration, the drawing figures illustrate the general manner of construction, and descriptions and details of well-known features and techniques may be omitted to avoid unnecessarily obscuring the discussion of the described embodiments of the invention. Additionally, elements in the drawing figures are not necessarily drawn to scale. For example, the dimensions of some of the elements in the figures may be exaggerated relative to other elements to help improve understanding of embodiments of the present invention. Certain figures may be shown in an idealized fashion in order to aid understanding, such as when structures are shown having straight lines, sharp angles, and/or parallel planes or the like that under real-world conditions would likely be significantly less symmetric and orderly. The same reference numerals in different figures denote the same elements, while similar reference numerals may, but do not necessarily, denote similar elements.

47. In addition, it should be apparent that the teaching herein can be embodied in a wide variety of forms and that any specific structure and/or function disclosed herein is merely representative. In particular, one skilled in the art will appreciate that an aspect disclosed herein can be implemented independently of any other aspects and that several aspects can be combined in various ways.

48. The present disclosure is described below with reference to functions, block diagrams and flowchart illustrations of the methods, apparatuses, and computer program according to one or more exemplary embodiments. Each described function, block of the block diagrams and flowchart illustrations can be implemented in hardware, software, firmware, middleware, microcode, or any suitable combination thereof. If implemented in software, the functions, blocks of the block diagrams and/or flowchart illustrations can be implemented by computer program instructions or software code, which may be stored or transmitted over a computer-readable medium, or loaded onto a general purpose computer, special purpose computer or other programmable data processing apparatus to produce a machine, such that the computer program instructions or software code which execute on the computer or other programmable data processing apparatus, create the means for implementing the functions described herein.

49. Embodiments of computer-readable media includes, but are not limited to, both computer storage media and communication media including any medium that facilitates transfer of a computer program from one place to another. As used herein, a "computer storage media" may be any physical media that can be accessed by a computer or a processor. In addition, the terms "memory" and "computer storage media" include any type of data storage device, such as, without limitation, a hard drive, a flash drive or other flash memory devices (e.g. memory keys, memory sticks, key drive), CD-ROMs or other optical data storage devices, DVDs, magnetic disk data storage devices or other magnetic data storage devices, data memory components, RAM, ROM and EEPROM memories, memory cards (smart cards), solid state drive (SSD) memories, and any other form of medium able to be used to transport or store or memorize data or data structures able to be read by a computer processor, or a combination thereof. Furthermore, various forms of computer-readable media may transmit or carry instructions to a computer, such as a router, a gateway, a server, or any data transmission equipment, whether this involves wired transmission (via coaxial cable, optical fibre, telephone wires, DSL cable or Ethernet cable), wireless transmission (via infrared, radio, cellular, microwaves) or virtualized transmission equipment (virtual router, virtual gateway, virtual tunnel end, virtual firewall). According to the embodiments, the instructions may comprise code in any computer programming language or computer program element, such as, without limitation, the languages of assembler, C, C++, Visual Basic, HyperText Markup Language (HTML), Extensible Markup Language (XML), HyperText Transfer Protocol (HTTP), Hypertext Preprocessor (PHP), SQL, MySQL, Java, JavaScript, JavaScript Object Notation (JSON), Python, and bash scripting.

50. Unless specifically stated otherwise, it will be appreciated that throughout the following description discussions utilizing terms such as processing, computing, calculating, determining, or the like, refer to the action or processes of a computer or computing system, or similar electronic computing device, that manipulate or transform data represented as physical, such as electronic, quantities within the registers or memories of the computing system into other data similarly represented as physical quantities within the memories, registers or other such information storage, transmission or display devices of the computing system.

51. The terms "comprise," "include," "have," and any variations thereof, are intended to cover a non-exclusive inclusion, such that a process, method, article, or apparatus that comprises a list of elements is not necessarily limited to those elements, but may include other elements not expressly listed or inherent to such process, method, article, or apparatus.

52. Additionally, the word "exemplary" is used herein to mean "serving as an example, instance, or illustration". Any embodiment or design described herein as "exemplary" is not necessarily to be construed as preferred or advantageous over other embodiments or designs. The terms "in particular", "for example", "example", "typically" are used in the present description to denote examples or illustrations of non-limiting embodiments that do not necessarily correspond to preferred or advantageous embodiments with respect to other possible aspects or embodiments.

53. The terms "operationally coupled", "coupled", and their various variants and forms used in the present description refer to couplings, that may be direct or indirect, and comprise in particular connections between electronic equipment or between portions of such equipment that allow operations and modes of operation as described in the present description. In addition, the term "coupled" is not limited to physical or mechanical connections or couplings. For example, an operational coupling may include one or more wired connection(s) and/or one or more wireless connection(s) between two or more items of equipment that allow simplex and/or duplex communication links between the equipment or portions of the equipment. According to another example, an operational coupling or a connection may include a wired-link and/or wireless coupling for allowing data communications between a server of the proposed system and another item of equipment of the system.

54. As used herein, the term "pixel" means a picture element or an elementary component of an image, a color component image, or a (luma or chroma) channel type image, and data corresponding thereto, which may be represented by a numeric value, e.g. a natural integer value. A pixel of a digital image may be coded digitally, and its value may be coded into a bitstream. Further, a pixel of an image may represent image data according to color spaces.

55. It is now referred to Figure 1.

56. Figure 1 shows a flowchart illustrating a proposed method for determining a standard plane according to one or more embodiments of the present subject disclosure.

57. As shown on Figure 1, in a step ROI_DETERMIN S10, at least one region of interest may be determined in a medical image IMG.

58. The at least one region of interest may comprise at least one element of interest. The elements of interest may depend on the medical imaging modality and/or the medical examination. For instance, for a fetal ultrasound image, the element of interest may be an element in the group comprising: a brain, a vessel, an orbit, a nose, a mouth, a cavity, an abdomen etc.

59. In one or more embodiments, the at least one region of interest may be manually determined on the image IMG.

60. Alternatively or in combination, in one or more embodiments, the at least one region of interest may be automatically determined, for instance using a machine learning algorithm. The machine learning algorithm may take the image IMG as input.

61. In one or more embodiments, the at least one region of interest of the image IMG may be determined using a deep learning algorithm. More specifically, the deep learning algorithm may be a convolutional neural network algorithm. In one or more exemplary embodiments, an algorithm from the YOLO family, such as YOLOv5, may be used. Documentation regarding the YOLO algorithms may for instance be found at https://docs.ultralytics.com/.

62. In one or more embodiments, training images may be used to train the machine learning algorithm to detect regions of interest comprising at least one element of interest. The training images may comprise images from the standard medical practice. The training images may be annotated, i.e a region of interest may be determined on the training image and associated with a label corresponding to the element of interest comprised in the region of interest, by a specialist. Typically, the specialist may be a medical expert such as a physician, a clinician, a radiologist, etc.

63. In one or more embodiments, the training images may be acquired with different apparatuses to increase the variability in the training images. Alternatively or in combination, the training images may be annotated by different specialists to increase the variability in the labels assigned to the elements of interest.

64. In one or more embodiments, the training images may be rescaled to have a common size. For instance, the common size, in pixels, may be comprised between 320 x 320 and 1280 x 1280. One example of usable common size is 640 x 640. In these exemplary embodiments, the medical image IMG given as input to the step ROI_DETERMIN S10 may also be rescaled to the common size.

65. In one or more embodiments, data augmentation may be applied to some of training images, and even in some embodiments to all the training images. Data augmentation may comprise one or several augmentation technics among a brightness augmentation, for instance Hue, Saturation and Value (HSV) augmentation, an affine transformation, a horizontal and vertical flip data augmentation.

66. In one or more embodiments, the machine learning algorithm may be trained using the COCO dataset. Documentation regarding the COCO dataset may for instance be found at https://cocodataset.org/#home.

67. In the step ROI_DETERMIN S10, for one or more elements of interest, or even for each element of interest, a probability that the element of interest is on the image IMG, may be obtained. For instance, the probability that the element of interest is on the image may be manually set. Alternatively, the probability that the element of interest is on the image may be obtained as an output of the machine learning algorithm.

68. In one or more embodiments, the determined regions of interest may be associated with a first confidence score. The first confidence score may be the probability that the element of interest comprised in the determined region of interest is on the image.

69. In a step CANDIDATE_DETERMIN S11, at least one candidate plane of the at least one region of interest determined in the step ROI_DETERMIN S10 may be determined. That is, the determined candidate plane depends on the region of interest determined in the step ROI_DETERMIN S10.

70. The candidate plane may be chosen in a predetermined group of reference planes. The group of reference planes may be specific to the medical imaging modality and/or to the medical examination.

71. In one or more embodiments, correspondences between the elements of interest and candidate planes may be established. For instance, an element of interest may be associated with one or more candidate planes. The correspondences may be specific to the medical imaging modality and/or to the medical examination. For instance, for the fetal ultrasound, the brain may be associated with, at least, the transthalamic view and the transcerebellar view.

72. In one or more embodiments, for the region of interest determined at step ROI_DETERMIN S10, the candidate plane associated with the element of interest comprised in the region of interest may be associated with the region of interest.

73. Alternatively or in combination, in one or more embodiments, when the region of interest is associated with a first confidence score, the determination of the candidate plane may depend on said first confidence score. For instance, a threshold of detection may be associated with the region of interest. The candidate plane may be determined if the first confidence score associated with the region of interest is higher than the threshold of detection.

74. Further, in one or more embodiments, the threshold of detection may be determined based on a validation dataset. For instance, the determined threshold of detection may be a threshold maximizing the sensitivity of the determination of the candidate plane over the validation dataset. Alternatively or in combination, the determined threshold of detection may be a threshold maximizing the F1-score of the determination of the candidate plane over the validation dataset.

75. Hence, at the end of the step CANDIDATE_DETERMIN S11, a group of candidate planes determined based on the regions of interest determined at step ROI_DETERMIN S10 may be obtained.

76. In a step ANAT_STRUCTUR_DETERMIN S12, at least one anatomical structure may be determined in the region of interest determined in the step ROI_DETERMIN S10 or in the medical image IMG.

77. In one or more embodiments, the at least one anatomical structure may be manually determined in the region of interest determined in the step ROI_DETERMIN S10 or in the image IMG.

78. Alternatively or in combination, in one or more embodiments, the at least one anatomical structure may be automatically determined, for instance using a machine learning algorithm. The machine learning algorithm may take the region of interest determined in the step ROI_DETERMIN S10 and/or the image IMG as input. Further, in one or more embodiments, the at least one anatomical structure may be determined using a deep learning algorithm. More specifically, the deep learning algorithm may be a convolutional neural network algorithm. In one or more exemplary embodiments, an algorithm from the YOLO family, such as YOLOv5, may be used.

79. Additionally, in one or more embodiments, the machine learning algorithm may be specific to an anatomical zone associated with anatomical structures to be possibly determined. That is, the machine learning algorithm specific to an anatomical zone may allow to detect the associated anatomical structures in the region of interest determined in the step ROI_DETERMIN S10 or in the medical image IMG given as input. The anatomical zone may be specific to the medical imaging modality and/or to the medical examination. For instance, for the fetal ultrasound, the anatomical zone may be chosen in the group comprising: the brain, the abdomen, and the heart. That is, for each anatomical zone a specific machine learning algorithm may be used.

80. As a non-limiting illustrative example, the brain anatomical zone may for instance comprise the following anatomical structures: a cavum septum pellucidum, a thalamus, a cerebellum etc. That is, the machine learning algorithm specific to the brain anatomical zone may allow to detect the cavum septum pellucidum, the thalamus, the cerebellum and the other anatomical structures of the brain anatomical zone in the region of interest determined in the step ROI_DETERMIN S10 or in the medical image IMG given as input.

81. In one or more embodiments, specific training images may be used to train each machine learning algorithm to detect the anatomical structures of the associated anatomical zone. The training images may comprise images from the standard medical practice. The training images may be annotated, *i.e* an anatomical structure of the associated anatomical zone may be determined on the training image and associated with a label corresponding to the anatomical structure, by a specialist. Typically, the specialist may be a medical expert such as a physician, a clinician, a radiologist, etc.

82. In one or more embodiments, the training images may be acquired with different apparatuses to increase the variability in the training images. Alternatively or in combination, the training images may be annotated by different specialists to increase the variability in the labels assigned to the anatomical structures of the anatomical zone.

83. In one or more embodiments, the training images may be rescaled to have a common size. For instance, the common size, in pixels, may be comprised between 320 x 320 and 1280 x 1280. One example of usable common size is 640 x 640. In these exemplary embodiments, the region of interest determined in the step ROI_DETERMIN S10 and/or the image IMG given as input to the step ANAT_STRUCTUR_DETERMIN S12 may also be rescaled to the common size.

84. In one or more embodiments, data augmentation may be applied to some of training images, and even in some embodiments to all the training images. Data augmentation may comprise one or several augmentation technics among a brightness augmentation, for instance the HSV augmentation, an affine transformation, a horizontal and vertical flip data augmentation.

85. In one or more embodiments, each machine learning algorithm may be pre-trained using the COCO dataset.

86. In one or more embodiments, in the step ANAT_STRUCTUR_DETERMIN S12, the region of interest determined in the step ROI_DETERMIN S10 and/or the image IMG may be given as input to each of the machine learning algorithms specific to an anatomical zone. That is, each of the machine learning algorithms specific to an anatomical zone may output whether an anatomical structure of the anatomical zone is detected in the region of interest determined in the step ROI_DETERMIN S10 and/or the image IMG given as input.

87. Alternatively, in one or more embodiments, correspondences between the candidate planes and the anatomical zone may be established. For instance, a candidate plane may be associated with one or more anatomical zone. The correspondences may be specific to the medical imaging modality and/or to the medical examination. For instance, for the fetal ultrasound, the transthalamic view, the transcerebellar view, the transventricular view, the upper lip nose and nostrils view, and the orbits view may be associated with the brain anatomical zone.

88. In one or more embodiments, in the step ANAT_STRUCTUR_DETERMIN S12, the region of interest determined in the step ROI_DETERMIN S10 and/or the image IMG may be given as input to the one or more machine learning algorithms specific to an anatomical zone associated with the candidate planes determined in the step CANDIDATE_DETERMIN S11. That is, only the machine learning algorithms specific to an anatomical zone associated with the candidate planes determined in the step CANDIDATE_DETERMIN S11 may be launched, allowing to increase the efficiency of the standard plane determination method by reducing the number of calculations.

89. Hence, at the end of the step ANAT_STRUCTUR_DETERMIN S12, a list of anatomical structures present in the region of interest determined in the step ROI_DETERMIN S10 and/or the image IMG may be obtained.

90. In a step STD_PLANE_DETERMIN S13, a candidate plane from the group of candidate planes determined at step CANDIDATE_DETERMIN S11 may be selected based on the presence or the absence of the anatomical structure determined at step ANAT_STRUCTUR_DETERMIN S12 in said candidate plane. Said selected candidate plane is designated as the standard plane for the medical image IMG.

91. That is, in the step STD_PLANE_DETERMIN S13, the anatomical structures' determination of the step ANAT_STRUCTUR_DETERMIN S12 may be used to correct errors of the candidate planes' determination of step CANDIDATE_DETERMIN S11. More specifically, consistency and/or contradiction between the candidate planes' determination of step CANDIDATE_DETERMIN S11 and the anatomical structures' determination of the step ANAT_STRUCTUR_DETERMIN S12 may be exploited in the step STD_PLANE_DETERMIN S13. For instance, a contradiction may be found when an anatomical structure determined at step ANAT_STRUCTUR_DETERMIN S12 may not be associated with the candidate plane determined at step CANDIDATE_DETERMIN S11.

92. In one or more embodiments, one or several, or even each, possible candidate plane of the step CANDIDATE_DETERMIN S11 may be associated, for instance through a correspondence table, with at least one possible anatomical structure of the step ANAT STRUCTUR_DETERMIN S12. A consistency may be found when the anatomical structure determined at step ANAT_STRUCTUR_DETERMIN S12 is associated with the candidate plane determined at step CANDIDATE_DETERMIN S11. A contradiction may be found when the anatomical structure determined at step ANAT_STRUCTUR_DETERMIN S12 is not associated with the candidate plane determined at step CANDIDATE_DETERMIN S11.

93. As a non-limiting illustrative example, the presence of a cerebellum in a transthalamic view may be contradictory. That is, the transthalamic view may not be associated with the cerebellum. Therefore, if the cerebellum is detected at step ANAT_STRUCTUR_DETERMIN S12 and the transthalamic view is determined at step CANDIDATE_DETERMIN S11, this may traduce an error either in the determination of the transthalamic view as a standard plane at step CANDIDATE_DETERMIN S11 and/or an error in the determination of the cerebellum as an anatomical structure in the step ANAT_STRUCTUR_DETERMIN S12.

94. Further, in one or more embodiments, an alert may be triggered when at least one contradiction is found between the candidate planes' determination of step CANDIDATE_DETERMIN S11 and the anatomical structures' determination of the step ANAT_STRUCTUR_DETERMIN S12. Said alert may warn the specialist about the contradiction. The specialist may hence correct either the standard plane determined at step CANDIDATE_DETERMIN S11 and/or the anatomical structure determined at step ANAT_STRUCTUR_DETERMIN S12.

95. As illustrated on Fig. 2, in or more embodiments, a proposed method for determining a standard plane may also comprise a colorimetry analysis COLOR_ANALYSIS S20 and/or a writing analysis WRITING_ANALYSIS S21. While both COLOR_ANALYSIS S20 and WRITING_ANALYSIS S21 steps are represented on Figure 2, it is to be noted that only one of the steps COLOR_ANALYSIS S20 and WRITING_ANALYSIS S21 may be implemented. Further, the order of the COLOR_ANALYSIS S20 and WRITING_ANALYSIS S21 steps may be changed.

96. In one or more embodiments, the medical image IMG' may be in the Digital imaging and communications in medicine (DICOM) format. That is, the medical image IMG' may comprise metadata in addition to the pixels. The metadata may comprise information regarding an apparatus that provided the medical image IMG'.

97. In one or more embodiments, in an optional step COLOR_ANALYSIS S20, colors of the pixels present in the medical image IMG' may be analyzed. Typically, ultrasound views may be in grayscale, except for 3D views, which may be in a nuance of orange, and Doppler views, for which blue and red colors may appear. Hence, the color analysis may allow to detect whether the medical image IMG' is a 3D view, a Doppler view or another ultrasound view.

98. Additionally, 3D views may have a mention "3D" written on the view. The position and the color of said mention may vary depending on the manufacturer of the apparatus that provided the 3D view. However, knowing said manufacturer, for example from the metadata, the position and the color of said mention may be constant. Hence, zooming on said position may allow to detect whether said mention is present or not. In particular, color of the pixels present at said position may be analyzed. If said color of said mention is detected at said position, the standard plane associated with the view may be a 3D view.

99. Therefore, based on the color analysis COLOR_ANALYSIS S20, the group of possible candidate planes for the step of the candidate planes' determination CANDIDATE_DETERMIN S11' may be reduced.

100. In one or more embodiments, in an optional step WRITING_ANALYSIS S21, writings in the medical image IMG' may be detected. Typically, on some views, writings may indicate the view taken. For instance, for the biparietal view (BIP), the CRL view, the femur view, the abdomen view and the eyes view, biometrics may be measured by the specialist. Said biometrics may be indicated, with their name, on the image IMG'. Hence, reading the writings on the image IMG' may allow to classify the image IMG', *i.e* to find the standard plane associated with the image IMG'. Additionally, the image IMG' may comprise writings such as a name of the view, for instance annotated by the specialist, and/or the name of anatomical structures. Said writings may provide indications on the standard plane associated with the medical image IMG'. Based on the writing analysis WRITING_ANALYSIS S21, it may therefore be possible to reduce the group of possible candidate planes for the step of the candidate planes' determination CANDIDATE_DETERMIN S11'.

101. In one or more embodiments, an optical character recognition (OCR) algorithm, such as Tesseract or EasyOCR, may be used for the writing analysis WRITING_ANALYSIS S21. Documentation regarding the EasyOCR algorithm may for instance be found at https://qithub.com/JaidedAl/EasyOCR.

102. Further, in one or more embodiments, the medical image IMG' may be preprocessed for the writing analysis WRITING_ANALYSIS S21. Typically, the writings in the image IMG' may depend on the apparatus that provided the medical image IMG'. For instance, a localization of the writings in the image IMG' may be manufacturer dependent. Hence, knowing, for example from the metadata, the manufacturer of the apparatus that provided the medical image IMG' may allow to directly perform the writing analysis in said localization. In particular, in one or more embodiments, the localization of the writings in the image IMG' may be automatically detected based on the metadata.

103. In a step ROI_DETERMIN S10', at least one region of interest may be determined in the medical image IMG' as explained in reference to the step ROI_DETERMIN S10 in Figure 1.

104. In a step CANDIDATE_DETERMIN S11', a group of candidate planes determined based on the regions of interest determined in step ROI_DETERMIN S10' may be obtained as explained in reference to the step CANDIDATE_DETERMIN S11 in Figure 1. Additionally, the group of candidate planes may be reduced based on the color analysis performed in the step COLOR_ANALYSIS S20 and/or the writing analysis performed in the step WRITING_ANALYSIS S21 as explained above.

105. In a step STD_PLANE_DETERMIN S13', a candidate plane from the group of candidate planes determined at step CANDIDATE_DETERMIN S11' may be selected based on the presence or the absence of the anatomical structure determined at step ANAT_STRUCTUR_DETERMIN S12' in said candidate plane as explained in reference to the step STD_PLANE_DETERMIN S13 in Figure 1.

106. As illustrated on Figure 3, in one or more embodiments, the standard plane determination may comprise one or more iterations of a selection loop for selecting the standard plane from the group of candidate planes.

107. In the example of Figure 3, in an optional step COLOR_ANALYSIS S20", a color analysis, similar to the one described in reference to the step COLOR_ANALYSIS S20 in Figure 2, may be performed on the medical image IMG".

108. In particular, the step COLOR_ANALYSIS S20" may determine in a step S200 if the medical image IMG" is a Doppler view or a 3D view. If the medical image IMG" is categorized as a Doppler view or a 3D view ("OK" output of the step S200), the determined standard plane is one of the Doppler view and the 3D view.

109. Conversely, if the medical image IMG" is not categorized as a Doppler view or a 3D view ("NOK" output of the step S200), in an optional step WRITING_ANALYSIS S21", a writing analysis, similar to the one described in reference to the step WRITING_ANALYSIS S21 in Figure 2, may be performed on the medical image IMG".

110. More specifically, in the example represented on Figure 3 in a step READ_WRITING S210, the possible writings in the medical image IMG" may be read using an OCR algorithm, such as Tesseract or EasyOCR.

111. If some writings are detected in the step READ_WRITING S210 ("OK" output of the step S211), the content of the writings may be analyzed in a step S212.

112. For instance, if biometrics are written on the medical image, the standard plane determined for the medical image IMG" may be determined based on said biometrics. For example, names of the biometrics written on the medical image IMG" may be standardized and associated with a standard plane. "BPD" and "HC" may be the names of biometrics associated with the Transthalamic view, "Cereb" may be the name of a biometric associated with the Transcerebellum view etc.

113. If the writings analyzed, for example a biometrics; is associated with a standard plane in the step S212 ("OK" output of the step S212), the standard plane for the medical image IMG" is the standard plane associated with the writing on the medical image IMG".

114. Conversely ("NOK" output of the step S212), in an optional step REDUCE_CANDIDATE_SET S214, the group of possible candidate planes for the step of the candidate planes' determination CANDIDATE_DETERMIN S11" may be reduced.

115. For instance, the specialist may annotate the medical image IMG". In particular, the specialist may annotate some or the anatomical structures present in the image, which may reduce the group of possible candidate planes for the step of the candidate planes' determination CANDIDATE_DETERMIN S11". As an illustrative example, if the term "kidney" is detected on the medical image IMG", the medical image IMG", if it is an image of a reference plane, may only be one of the axial view of the two kidneys and the sagittal view of one of the two kidneys. That is, the group of possible candidate planes for said medical image IMG" may be reduced to two candidates: the axial view of the two kidneys and the sagittal view of one of the two kidneys.

116. In a step ROI_DETERMIN S10", at least one region of interest may be determined in the medical image IMG" as explained in reference to the steps ROI_DETERMIN S10, S10" in Figures 1 and 2.

117. In one or more embodiments, no region of interest may be determined in the medical image IMG". For instance, when the first confidence score is lower than the detection threshold, no region of interest may be determined in the medical image IMG". This may indicate that the medical image IMG" was erroneously acquired and is not part of the medical examination. In one or more embodiments, the specialist may be presented the medical image IMG" and proposed to confirm whether the medical image was erroneously acquired. If not, the specialist may be proposed to associate the medical image IMG" with its standard plane and/or with its anatomical structure.

118. For one or several, or even for all, regions of interest determined in the step ROI_DETERMIN S10", a candidate plane of said region of interest may be determined in a step CANDIDATE_DETERMIN S11" as explained in reference to the steps CANDIDATE_DETERMIN S11, S10' in Figures 1 and 2.

119. The group of candidate planes may comprise zero candidate, for instance in the variants where the regions of interest are associated with a first confidence score and a threshold of detection higher than the first confidence score. Alternatively, the group of candidate planes may comprise one or more candidate planes. In particular, the group of candidate planes may comprise candidate planes associated with different regions of interest when several regions of interest are determined at step ROI_DETERMIN S10".

120. In a step S30, the number of candidate planes in the group of candidate planes is evaluated. If the number of candidate planes is equal to zero ("NB=0" output of the step S30), it is determined in a step S31 that the medical image IMG" is not in the scope of the reference planes for said medical examination. This may indicate that the medical image IMG" was erroneously acquired and is not part of the medical examination. In one or more embodiments, the specialist may be presented the medical image IMG" and proposed to confirm whether the medical image was erroneously acquired. If not, the specialist may be proposed to associate the medical image IMG" with its standard plane and/or with its anatomical structure.

121. Alternatively, if the number of candidate planes is greater than one ("NB>1" output of the step S30), the candidate planes in the group of candidate planes may be ranked in a step CANDIDATE_RANK S32. The candidates planes may be ranked from the more probable to the less probable.

122. In this regard, in one or more embodiments, one or several, or even each; candidate plane from the group of candidate planes may be associated with a priority level and/or a second confidence score. The candidate planes may be ranked based on the priority levels and/or the second confidence scores.

123. The priority levels may be for instance defined in a table of priority based in a *priori* knowledge. Typically, in a convention, the higher is the probability that the candidate plane reflects the reference plane imaged in the medical image IMG", the higher is the priority level. In this convention, the candidate plane with the highest priority level may be ranked as the most probable. Other conventions may be chosen.

124. The second confidence scores may be the first confidence score determined in the step ROI_DETERMIN S10".

125. In the cases where two candidate planes are associated with the same priority level, the candidate with a higher second confidence may be assigned a higher rank than the candidate with the same priority level but a lower second confidence score.

126. In the example of Figure 3, for each candidate plane, in a step ANAT_STRUCTUR_DETERMIN S12", at least one anatomical structure is determined in the region of interest associated with the candidate plane as explained in reference to the steps ANAT_STRUCTUR_DETERMIN S12, S12' in Figures 1 and 2.

127. The step ANAT_STRUCTUR_DETERMIN S12" represented on Figure 3 is executed outside of the selection loop performed in a step STD_PLANE_DETERMIN S13". Alternatively, the step ANAT_STRUCTUR_DETERMIN S12" may be executed in the selection loop. That is, for a current candidate plane CANDIDATE_k of the selection loop, at least one anatomical structure may be determined in the region of interest associated with the current candidate plane CANDIDATE_k.

128. In some embodiments, no structure may be determined in the step ANAT_STRUCTUR_DETERMIN S12". In these cases, it may be determined at the end of the step ANAT_STRUCTUR_DETERMIN S12", that the image IMG" is out of the scope of the reference planes for the medical examination.

129. In some embodiments, at least two candidate planes CANDIDATE_k and CANDIDATE_k' are associated with two distinct anatomical zones. In these cases, a structure determination for the two anatomical zones may be performed in the step ANAT_STRUCTUR_DETERMIN S12". In the cases where only anatomical structures associated with one of the two anatomical zones (for instance the anatomical zone associated with CANDIDATE_k') are determined, the candidate plane associated with said anatomical zone is determined as the standard plane (CANDIDATE_k' in this example).

130. Alternatively, in a step STD_PLANE_DETERMIN S13", a candidate plane from the group of candidate planes determined at step CANDIDATE_DETERMIN S11" may be selected based on the presence or the absence of the anatomical structure determined at step ANAT_STRUCTUR_DETERMIN S12" in said candidate plane CANDIDATE_k as explained in reference to the step STD_PLANE_DETERMIN S13, S13' in Figures 1 and 2.

131. More specifically, the selection loop may be initialized in a step S130 with a value of a loop variable k equal to one by convention. In this convention, the candidate plane CANDIDATE_1 for k =1, is the candidate plane ranked as the most probable in the step CANDIDATE_RANK S32. The candidate plane CANDIDATE_NB for k =NB, where NB is the number of candidate planes determined at step S30, is the candidate plane ranked as the less probable in the step CANDIDATE_RANK S32.

132. In a step S131, a compatibility of the candidate plane CANDIDATE_k with the anatomical structures determined at step S12" may be determined. If the candidate plane CANDIDATE_k is compatible with the anatomical structures determined at step S12" ("OK" output of the step S131), the candidate plane CANDIDATE_k is determined as the standard plane in a step S132 and may be returned in a step S133.

133. In some embodiments, the anatomical structures determined at step S12" may not be directly compatible with the candidate plane CANDIDATE_k. However, the anatomical structures determined at step S12" may be compatible with another candidate plane CANDIDATE_k' associated the same anatomical zone as the candidate plane CANDIDATE_k. In this situation, in some embodiments, the other candidate plane CANDIDATE_k' may be determined as the standard plane.

134. Conversely, if an incompatibility between the candidate plane CANDIDATE_k and the candidate plane CANDIDATE_k is incompatible with the anatomical structures determined at step S12" ("NOK" output of the step S131), the loop variable k may be incremented by one in a step S136, if the loop variable k is smaller than NB ("NOK" output of the step S134).

135. If the loop variable k is equal to NB ("OK" output of the step S134), it is determined in a step S135 that the image IMG" is out of the scope of the reference planes for the medical examination.

136. In some non-represented embodiments, a plurality of planes may be selected based on the presence or the absence of the anatomical structure in the candidate plane. For instance, in one or more embodiments, a third confidence score may be associated with each candidate plane. Said third confidence score may be a confidence score set by the specialist at the end of the step ANAT_STRUCTURE_DETERMIN S12, S12', S12" or may be a confidence score provided by the machine learning algorithm used in the step ANAT_STRUCTURE_DETERMIN S12 when the region of interest associated with the candidate plane is given as input. Said third confidence score may be compared to a threshold range comprised for instance between 0.1 and 0.9. One example threshold value is 0.3. It is also apparent that at least one third confidence score can be obtained for at least one anatomical structure respectively. Stated otherwise, a third confidence score can be obtained for each anatomical structure in the image. Thus, a plurality of third confidence scores may be obtained for a plurality of anatomical structures respectively.

137. For two or more, preferably three, candidate planes CANDIDATE_k of a plurality of candidate planes having the associated confidence score comprised within the threshold range and a compatibility with the anatomical structures determined at step S12", the candidate planes CANDIDATE_k may be displayed, for instance on a screen.

138. The standard plane may be selected among the displayed candidate planes. For instance, the specialist may select the standard plane among the displayed candidate planes.

139. Information on said selected standard plane may be received and stored in a database. Said database may be used to train the standard plane determination for a further image. Hence, the robustness of the standard plane determination may be increased.

140. The at least third confidence score may also serve to select an output image among a plurality of medical images. Indeed, the at least one third confidence score provides an indication of the confidence of detection of each anatomical structure, and it may be desirable to output the image that is associated with the highest confidence of detection of at least one given anatomical structure.

141. An example of such selection is a fetal ultrasound which is performed by a non-expert, who is not expected to be able to perform the fetal ultrasound from the right point of view. In order to nevertheless obtain a suitable image, the non-expert user can be asked to perform large movements that cause the creation of a video (or series of successive images) of fetal ultrasound images from many points of views, then a single image which is expected to be the best image (i.e the image that best represent at least one reference anatomical structure) is selected based on at least one third confidence score associated with the at least one reference anatomical structure.

142. Therefore, the non-expert user can capture many images, and it can be expected that at least one "best" image among the many images accurately represents at least one anatomical structure that is sought, and this best image can be selected as the output image based on the at least one third confidence score.

143. After the selection of the output image, the output image can be displayed. For example, if the plurality of medical images represent a fetal ultrasound, the output image can be displayed to an obstetrician.

144. In some cases, no anatomical structure is detected. It is for example the case if a fetal ultrasound which has to access Crown-Rump-Length have been captured (View model), but no anatomical structure model to check the presence of standard structure on this image. In such cases, the image which exhibits the highest first confidence score (e.g the highest confidence of the view model) can be selected.

145. Thus, in absence of detection of an anatomical structure, the image which is expected to provide the best view is selected.

146. In other examples, the images are expected to represent a single anatomical structure. For example, a fetal ultrasound may be focused on one particular anatomical structures of the brain only.

147. If a single reference anatomical structures is looked after, the output image can be selected by:
- selecting a subset of the plurality of images where the single reference structure is present;
- selecting the output image as the image in the subset for which the third confidence score of the single reference anatomical structure is the highest.

148. Thus, the output image is in this case the image where the single reference anatomical structure is present with the highest third confidence score, i.e the image for which the confidence that the single reference anatomical structure is present is the highest.

149. In yet other cases, a plurality of reference anatomical structures can be looked after. For example for the 3 vessels View of fetal heart ultrasound the practitioner should exhibit several anatomical structures among them, superior vena cava, aorta and pulmonary artery

150. In such cases, a plurality of third confidence scores may be computed for a plurality of reference anatomical structures respectively, and a weighted third confidence score can be computed as a weighted average of the plurality of third confidence scores.

151. For example, if a fetal ultrasound represents the 3 vessels View of the fetal heart: brain and the heart:
- a third confidence score can be computed for the superior vena cava ;
- a third confidence score can be computed for the aorta ;
- a third confidence score can be computed for the pulmonary artery ;
- a weighted third confidence score can be computed as a weighted average of the third confidence score for the pulmonary artery, the third confidence score for the aorta and the third confidence score for the superior vena cava.

152. If all the anatomical structures are of equal importance, the weighted average is an average. In the former example, the weighted average may be the sum of a third of the third confidence score for the pulmonary artery, a third of the third confidence score for the superior vena cava and a third of the third confidence score of the aorta.

153. Otherwise, if the anatomical structure are not of equal importance, the weighted average may give more weight to some of the anatomical structures. For example, if the confidence regarding the superior vena cava is more importance than the confidence regarding the pulmonary artery, the weighted average may give more weight to the third confidence score of the pulmonary artery. For example, the weighted third confidence score may be a weighted average of 2/3 of the third confidence score of the superior vena cava, and 1/6 of the third confidence score of the pulmonary artery and 1/6 of the third confidence score of the aorta.

154. The output image can then be selected as the image that maximizes the weighted third confidence score, and thus as the image that provides the best overall representation of the plurality of reference anatomical structures.

155. In a number of embodiments of the invention, the output images is selected by:
- first, selecting a subset of said plurality of medical images where the highest number of reference anatomical structures are present. For example, if 3 reference anatomical structures are looked after, the subset may comprise:
   ∘ all the images that comprise the 3 reference anatomical structures, if at least one image comprises the 3 reference anatomical structures ;
   ∘ otherwise, all the images that comprise 2 of the 3 reference anatomical structures, if at least one image comprises 2 the 3 reference anatomical structures ;
   ∘ etc.
- then, selecting said output image as the image of said subset that has the highest weighted third confidence score.

156. Thus, the output image can be selected as one of the images that contains the highest number of the reference anatomical structures, and, among these images, the one that has the highest confidence score associated with the representation of the reference anatomical structures.

157. The selection of the output image has been described above with reference to an example of a selection of an output image in a video representing successive images of a fetal ultrasound. The selection of the output image is however not restricted to this example. It can be more generally applied to other examples of videos that represent successive medical images performed by a same medical device from different point of views, but also to different images from different sources. For example, the method of selection can be applied to select the best image among images captured by a plurality of different obstetricians respectively.

158. Figure 4 shows an example of a standard plane STD_PLANE associated with the image IMG and determined with a method of the present disclosure.

159. In the example of Figure 4, the determined standard plane is the transthalamic view, which is symbolized in Figure 4 by the pictogram STD_PLANE. The standard plane may have been determined using a method of the present disclosure exploiting the writings WRITING in the image.

160. Figure 5 shows an example of a standard plane determined with a method of the present disclosure.

161. In the example of Figure 5, two regions of interest ROI_1 , ROI_2 are determined in the image IMG. The first region of interest ROI_1 comprises the brain as element of interest. The second region of interest ROI_2 comprises a hand as element of interest. The candidate plane associated with the first region of interest ROI_1 is the transthalamic view. The candidate plane associated with the second region of interest ROI_1 is the hand view. In this example, the transthalamic view has a higher priority than the hand view. Hence, the determined standard plane is the transthalamic view.

162. Figure 6 shows an example of candidate planes and of standard planes determined with a method of the present disclosure.

163. In the example of Figure 6, standard planes may have been determined for the images 600, 601, 602, 603, 604. In this example, images 600, 601, 602, 603, 604 replace the pictograms symbolizing the associated standard plane. In contrast, there is no medical image associated with the candidate planes symbolized by the pictograms 610, 611, 612, 613. This lack of medical images may for instance result from an oversight on the specialist's part.

164. Hence, advantageously, a checklist of the determined standard planes may be provided with the present invention. During the medical examination, the specialist may therefore easily check which standard planes have already been required and which remain to be acquired.

165. Part of these flow charts (Figures 1 to 3) can represent steps of an example of a computer program which may be executed by the device of Figure 7.

166. Figure 7 is a block diagram illustrating an embodiment of an apparatus according to the present disclosure.

167. In one or more embodiments, the device 70 may comprise a computer 701, said computer 701 comprising a memory 705 to store program instructions loadable into a circuit and adapted to cause circuit 704 to carry out the steps of the present invention when the program instructions are run by the circuit 704.

168. The memory 705 may also store data and useful information for carrying the steps of the present invention as described above.

169. The circuit 704 may be for instance:
- a processor or a processing unit adapted to interpret instructions in a computer language, the processor or the processing unit may comprise, may be associated with or be attached to a memory comprising the instructions, or
- the association of a processor / processing unit and a memory, the processor or the processing unit adapted to interpret instructions in a computer language, the memory comprising said instructions, or
- an electronic card wherein the steps of the invention are described within silicon, or
- a programmable electronic chip such as a FPGA chip (for « Field-Programmable Gate Array »).

170. The computer 701 may comprise an input interface 703 for the reception of images IMG, IMG', used for the above method according to the invention and an output interface 706 for providing the determined standard plane.

171. To ease the interaction with the computer, a screen 701 and a keyboard 702 may be provided and connected to the computer circuit 704.

172. Alternatively or in combination, the device 70 may be operatively coupled to at least one computer server. The at least one computer server may comprise a memory to store program instructions loadable into a circuit and adapted to cause circuit to carry out the steps of the present invention when the program instructions are run by the circuit.

173. The memory may also store data and useful information for carrying the steps of the present invention as described above.

174. The circuit may be for instance:
- a processor or a processing unit adapted to interpret instructions in a computer language, the processor or the processing unit may comprise, may be associated with or be attached to a memory comprising the instructions, or
- the association of a processor / processing unit and a memory, the processor or the processing unit adapted to interpret instructions in a computer language, the memory comprising said instructions, or
- an electronic card wherein the steps of the invention are described within silicon, or
- a programmable electronic chip such as a FPGA chip (for « Field-Programmable Gate Array »).

175. That is, in one or more embodiments, the standard plane for the medical image IMG, IMG' may be determined on the computer server. The result of the standard plane determination may be provided to the device 70 through the input interface 703. In particular, the result of the standard plane determination may be accessible through an application. In one or more embodiments, the application may be accessible via the Internet.While the invention has been described with respect to preferred embodiments, those skilled in the art will readily appreciate that various changes and/or modifications can be made to the invention without departing from the scope of the present subject disclosure as defined by the appended claims.

176. Although this invention has been disclosed in the context of certain preferred embodiments, it should be understood that certain advantages, features and aspects of the systems, devices, and methods may be realized in a variety of other embodiments. Additionally, it is contemplated that various aspects and features described herein can be practiced separately, combined together, or substituted for one another, and that a variety of combination and sub-combinations of the features and aspects can be made and still fall within the scope of the invention. Furthermore, the systems and devices described above need not include all of the modules and functions described in the preferred embodiments.

177. Information and signals described herein can be represented using any of a variety of different technologies and techniques. For example, data, instructions, commands, information, signals, bits, symbols, and chips can be represented by voltages, currents, electromagnetic waves, magnetic fields or particles, optical fields or particles, or any combination thereof.

178. Depending on the embodiment, certain acts, events, or functions of any of the methods described herein can be performed in a different sequence, may be added, merged, or left out all together (e.g., not all described acts or events are necessary for the practice of the method). Moreover, in certain embodiments, acts or events may be performed concurrently rather than sequentially.

179. This disclosure is not limited to the fetal ultrasound images described here, which are only examples. The invention encompasses every alternative that a person skilled in the art would envisage when reading this text. In particular, what has been exemplified above for fetal ultrasound images is applicable also for any medical image, including ultrasound images, computed tomography images, magnetic resonance images, etc., except for the features of the method and apparatus which are specific to fetal ultrasound images.

## Claims

1. Method for determining a standard plane in a medical image (IMG), said method comprising:
- determining (S10) at least one region of interest in the medical image,
- determining (S11) at least one candidate plane of said region of interest,
- determining (S12) at least one anatomical structure in the region of interest or in the medical image,
- selecting (S13) a plane from the group of said candidate planes based on the presence or the absence of said anatomical structure in said candidate plane, said selected plane being the standard plane.

2. Method according to claim 1, wherein the region of interest is associated with a first confidence score and the determination of the candidate plane of said region of interest depends on said first confidence score.

3. Method according to one of the preceding claims, wherein each candidate plane from the group of said candidate planes is associated with a priority level and/or a second confidence score, the method further comprising:
- ranking (S32) the candidate planes based on the priority levels and/or the second confidence scores,
wherein the selection of the plane from the group is based on the candidate planes' rank order.

4. Method according to any one of the preceding claims, said method further comprising:
- detecting (S210) writings in the region of interest or in the medical image,
- reducing (S214) the group of candidate planes based on said detected writings.

5. Method according to any of the preceding claims, said method further comprising:
- determining an apparatus that provided the medical image,
- reducing the group of candidate planes based on said determined apparatus.

6. Method according to claims 4 and 5, said method further comprising: detecting (S210), based on the determined apparatus, the writings in the region of interest or in the medical image.

7. Method according to any one of the preceding claims, said method further comprising:
- analyzing (S20) colors of pixels present in the region of interest or in the medical image,
- reducing the group of candidate planes based on said colors' analysis.

8. Method according to any one of the preceding claims, said method further comprising:
- selecting a plurality of planes based on the presence or the absence of the anatomical structure in the candidate plane,
- displaying the plurality of selected planes,
- receiving information on the selected plane corresponding to the standard plane,
- storing received information in a database, said database being used to train the standard plane determination.

9. Method according to any of the preceding claims, further comprising respectively associating to said at least one anatomical structure at least one third confidence score.

10. Method according to the preceding claims, comprising selecting, among a plurality of medical images, an output image based on said at least one third confidence score.

11. Method according on claim 10, comprising:
- computing a plurality of third confidence scores for a plurality of reference anatomical structures respectively;
- computing a weighted third confidence score as a weighted average of said plurality of third confidence scores.

12. Method according to the preceding claim, comprising:
- selecting a subset of said plurality of medical images where the highest number of reference anatomical structures are present;
- selecting said output image as the image of said subset that has the highest weighted third confidence score.

13. Computer software comprising instructions to implement at least a part of a method according to one of claims 1 to 12 when the software is executed by a processor (704).

14. Computer-readable non-transient recording medium on which a software is registered to implement a method according to one of claims 1 to 12 when the software is executed by a processor (704).
